# EUROPÄISCHE PATENTSCHRIFT

(11) **EP 4 346 627 B1**
(45) Veröffentlichungstag und Bekanntmachung des Hinweises auf die Patenterteilung: **15.10.2025**
(21) Anmeldenummer: 22732949.7
(22) Anmeldetag: 01.06.2022
(51) Int. Cl.: A61B 17/02, A61B 17/128, A61B 17/08, A61B 90/00, A61B 17/122

(54) **CHIRURGISCHE VORRICHTUNG ZUR VERKLEMMUNG IN GEWEBE**
SURGICAL DEVICE FOR CLAMPING IN TISSUE
DISPOSITIF CHIRURGICAL PERMETTANT UN MAINTIEN DANS UN TISSU

(30) Priorität: 02.06.2021 DE 102021114299
(43) Veröffentlichungstag der Anmeldung: 10.04.2024
(73) Patentinhaber: Eberhard Karls Universität Tübingen, Medizinische Fakultät, 72074 Tübingen (DE)
(72) Erfinder: HOFFMANN, Sascha, 72074 Tübingen (DE)
(74) Vertreter: Witte, Weller & Partner Patentanwälte mbB
(86) Internationale Anmeldenummer: PCT/EP2022/064952
(87) Internationale Veröffentlichungsnummer: WO 2022/253914

(56) Entgegenhaltungen:
- WO-A1-2015/087715
- CN-A- 111 759 472
- US-A1- 2007 106 314
- US-A1- 2009 198 107
- US-A1- 2010 204 727
- US-A1- 2017 035 404

## Beschreibung

Die vorliegende Erfindung betrifft eine chirurgische Vorrichtung zur Verklemmung in Gewebe, die innerhalb einer minimalinvasiven oder offen chirurgischen Behandlung zum Selbsthalten von Gewebe eingesetzt werden kann.

Bei einem operativen bzw. chirurgischen Eingriff zum Zweck der Therapie oder Diagnostik unterscheidet man zwischen einem offenen, chirurgischen Eingriff und einer minimalinvasiven Operation. Beide Eingriffe benötigen eine Vielzahl an Instrumenten bzw. Werkzeugen.

Bei der herkömmlichen offenen Chirurgie im Inneren des menschlichen Körpers muss der Chirurg einen hinreichend großen Einschnitt durchführen, um den Zugriff auf den interessierenden Bereich vornehmen zu können und um eine direkte Sichtlinie auf den Bereich zu ermöglichen. Einhergehend mit der Entwicklung von Endoskopen, chirurgischen Werkzeugen, die von außerhalb des Körpers des Patienten her gehandhabt werden können, und verschiedener Abbildungstechniken wie beispielsweise Ultraschallabbildung, Computertomographie und Magnetresonanzabbildung, ist es jedoch möglich geworden, chirurgische Eingriffe durch sehr kleine Einschnitte oder Körperöffnungen hindurch auszuführen, deren Größe für die traditionelle offene Chirurgie ungeeignet wäre. Derartige chirurgische Eingriffe werden generell als minimalinvasive Chirurgie bezeichnet.

Die minimalinvasive Chirurgie ist in den Fällen, in denen sie anwendbar ist, gegenüber dem Patienten sehr vorteilhaft, da sie ein geringeres Trauma verursacht als die offene Chirurgie. Oft führt sie auch zu einer Senkung der Behandlungskosten und zu einer Verkürzung der Krankenhausaufenthalte. Die herkömmlichen minimalinvasiven chirurgischen Vorrichtungen sind jedoch mit mehreren Nachteilen behaftet. Ein Nachteil besteht in einer leichten Ermüdbarkeit des Chirurgen aufgrund des Erfordernisses, die minimalinvasive chirurgische Vorrichtung während ihrer Verwendung manuell zu halten. Ein weiterer Nachteil besteht darin, dass die minimalinvasiven chirurgischen Vorrichtungen dem Chirurgen abverlangen, seine Hände in einer sehr unbequemen Position zu halten. Zudem können herkömmliche minimalinvasive chirurgische Vorrichtungen eine winklige Vergrößerung von Fehlbewegungen verursachen. Folglich kann der Chirurg eine Operation bei Verwendung minimalinvasiver chirurgischer Vorrichtungen nur mit beträchtlich weniger Handhabungspräzision und Präzision durchführen als bei Durchführung einer Operation mittels herkömmlicher Techniken, bei denen der Chirurg das Werkzeug direkt erfasst. Deshalb findet die minimalinvasive Chirurgie nur bei denjenigen chirurgischen Eingriffen Anwendung, die ein geringes Maß an Handhabungspräzision vom Chirurgen verlangen.

Eine typische chirurgische Vorrichtung zur Verwendung bei der minimalinvasiven Chirurgie weist ein längliches Rohr mit einem ersten Ende, das über einen Einschnitt oder eine Öffnung in den Körper des Patienten einführbar ist, und mit einem zweiten Ende auf, das sich aus dem Körper des Patienten heraus erstreckt und das vom Chirurgen ergriffen wird. Das erste Ende ist mit einem chirurgischen Werkzeug bestückt, während das zweite Ende mit einem Griff oder einem anderen Teil versehen ist, der vom Chirurgen ergriffen wird und der durch die Mitte des Rohrs mechanisch mit dem Werkzeug verbunden ist. Der Chirurg kann durch Handhaben des Griffs das Werkzeug betätigen, und er kann die Position des Werkzeugs verändern, indem er die Ausrichtung des Rohrs relativ zum Körper des Patienten verändert.

Gattungsgemäße medizinische Instrumente/Werkzeuge werden in der Praxis häufig als Greif-, Halte- und/oder Schneidwerkzeuge eingesetzt. So können die Maulteile Schneiden aufweisen, um Gewebe abzutrennen, oder stumpfe Flächen aufweisen, um beispielsweise abgetrenntes Gewebe zu halten, oder Blutgefäße abzuklemmen.

Zum Einbringen von chirurgischen Implantaten in Körperhöhlen werden heute in der minimalinvasiven Chirurgie häufig Trokare verwendet. Solche Trokare werden beispielsweise bei laparoskopischen und thoraskopischen Eingriffen eingesetzt. Durch die von außerhalb des Körpers in den Operationsraum führenden hülsenförmigen Trokaren können Instrumente und Gegenstände in die Körperhöhle eingeführt werden. Nachteilig hierbei ist, dass jedes Instrument durch einen einzelnen Trokar bzw. durch einen einzelnen Tubus gelegt werden muss. Demnach ist die Anzahl der Instrumente stets durch die Anzahl der Zugänge bzw. der Anzahl der Trokare beschränkt.

Die US 2010/0204727 A1, US 2009/0198107 A1, und die WO 2015/087715 A1 offenbaren chirurgische Vorrichtungen mit Klemmen, die in Operationen zum Halten von Gewebe, Organen oder Blutgefäßen eingesetzt werden

Sowohl für die offenen, chirurgischen Eingriffe als auch für die minimalinvasiven Operationen ist das Bedürfnis an der Entwicklung verbesserter, chirurgischer Werkzeuge nach wie vor sehr groß.

Vor diesem Hintergrund ist es eine Aufgabe dieser Erfindung, eine chirurgische Vorrichtung bereitzustellen, die es einem zu behandelnden Arzt erlaubt, einen medizinischen Eingriff vereinfacht durchzuführen, wobei die vorstehenden Nachteile überwunden werden sollen.

Erfindungsgemäß wird diese Aufgabe gelöst durch eine chirurgische Vorrichtung zur Verklemmung in Gewebe, wobei die chirurgische Vorrichtung Folgendes aufweist:
- eine erste Klemme mit zwei Klemmschenkeln und einer die Klemmschenkel zusammenhaltende, gelenkartig gelagerte Feder, wobei die erste Klemme einen distalen Klemmabschnitt aufweist, der zum Einklemmen und Halten von Gewebe ausgebildet ist, und einem proximalen Betätigungsabschnitt, der zum Öffnen und Schließen des Klemmabschnitts der ersten Klemme ausgebildet ist,
- eine zweite Klemme mit zwei Klemmschenkeln und einer die Klemmschenkel zusammenhaltende, gelenkartig gelagerte Feder, wobei die zweite Klemme einen distalen Klemmabschnitt aufweist, der zum Einklemmen und Halten von Gewebe ausgebildet ist, und einen proximalen Betätigungsabschnitt, der zum Öffnen und Schließen des distalen Klemmabschnitts der zweiten Klemme ausgebildet ist, und
- ein längliches formstabiles Verbindungsstück zum Verbinden der ersten und zweiten Klemme, wobei das Verbindungsstück am proximalen Betätigungsabschnitt der ersten Klemme und am proximalen Betätigungsabschnitt der zweiten Klemme zur Ausbildung eines stabilen Abstands zwischen der ersten Klemme und der zweiten Klemme fixiert ist.

Hierein offenbart, aber nicht beansprucht, ist ferner die Verwendung der erfindungsgemäßen, chirurgischen Vorrichtung innerhalb einer minimalinvasiven oder offen chirurgischen Behandlung, insbesondere innerhalb einer Biopsie/Endoskopie zum Selbsthalten von Gewebe.

Die der Erfindung zugrundeliegende Aufgabe wird auf diese Weise vollkommen gelöst.

Die vorliegende Erfindung stellt eine chirurgische Vorrichtung zur Verklemmung in Gewebe bereit, wobei die Vorrichtung für chirurgische Eingriffe und insbesondere für minimalinvasive chirurgische Eingriffe geeignet ist. Bei den Eingriffen kann es sich sowohl um einen diagnostischen oder therapeutisch medizinischen Vorgang handeln.

Mit der erfindungsgemäßen chirurgischen Vorrichtung wird ein medizinisches Werkzeug bereitgestellt, mit dem ein zu behandelnder Arzt in der Lage ist, während eines chirurgischen Eingriffs, Gewebe zu verklemmen und dieses während eines Eingriffs beispielsweise in Form bzw. auf Abstand zu halten.

Die erfindungsgemäße chirurgische Vorrichtung bietet ferner den Vorteil, dass sie Trokar bzw. Tubus unabhängig ist. Das bedeutet, dass sie zwar über einen Trokar zur behandelnden Stelle geführt werden kann, dort aber ohne Trokar verwendet werden kann, sodass in eine Körperöffnung mehrere erfindungsgemäße chirurgische Vorrichtungen durch nur einen einzelnen Tubus eingebracht werden können.

Die erfindungsgemäße Vorrichtung bzw. die erste und zweite Klemme kann durch Druckausübung innerhalb des proximalen Betätigungsabschnitts der jeweiligen Klemme geöffnet werden. Die Druckausübung kann beispielsweise durch direkten Fingerdruck des zu behandelnden Arztes oder durch ein weiteres Werkzeug, z.B. eine chirurgische Pinzette oder endoskopischen Klammer, erfolgen.

Mit der erfindungsgemäßen chirurgischen Vorrichtung wird darüber hinaus ein medizinisches Werkzeug bereitgestellt, welches sich ebenfalls in der Roboter-unterstützenden Chirurgie und Endoskopie einsetzen lässt. Hierbei bietet die Medizinrobotik den Vorteil, hochpräzise Bewegungen und damit die Handhabung der erfindungsgemäßen Vorrichtung Roboter-assistiert durchzuführen. In diesem Fall kann die erfindungsgemäße Vorrichtung bzw. die erste und zweite Klemme durch Druckausübung innerhalb des proximalen Betätigungsabschnitts der jeweiligen Klemme geöffnet werden. Die Druckausübung kann hierbei durch den Roboter selbst, bzw. einem Greifarm des Roboters erfolgen, wobei der Roboter beispielsweise mittels eines Joysticks vom zu behandelnden Arzt gesteuert wird.

Erfindungsgemäß wird unter einer "Klemme" eine Vorrichtung verstanden, mit der es einem zu behandelnden Arzt möglich ist, Gewebe einzuklemmen oder auf Abstand zu halten. Die erfindungsgemäßen Klemmen bzw. deren distaler Klemmabschnitt sind derart ausgestaltet, dass sie das Gewebe während des Eingriffs möglichst nicht verletzen bzw. ruptieren.

Erfindungsgemäß ist die erste als auch die zweite Klemme derart ausgestaltet, dass sie jeweils zwei Klemmschenkel aufweist, die durch eine gelenkartig gelagerte Feder zusammengehalten wird. Die beiden Klemmschenkel bilden einen distalen Klemmabschnitt und einen proximalen Betätigungsabschnitt aus. Der distale Klemmabschnitt wird insbesondere durch distale Abschnitte der beiden Klemmschenkel ausgebildet. Umgekehrt wird der proximale Betätigungsabschnitt durch proximale Abschnitte der beiden Klemmschenkel ausgebildet. Mit anderen Worten erstrecken sich die jeweiligen Klemmschenkel der ersten und zweiten Klemme vom jeweiligen distalen Klemmabschnitt bis hin zum jeweiligen Betätigungsabschnitt, wobei sie durch die jeweilige Feder gelenkartig zusammengehalten werden.

Gemäß einer Ausführungsform der vorliegenden Erfindung kann es sich bei der ersten und zweiten Klemme um identisch ausgestaltete Klemmen handeln. Hierbei können insbesondere der distale Klemmabschnitt der ersten und zweiten Klemme bzw. die distalen Abschnitte der zwei Klemmschenkel der ersten bzw. zweiten Klemme identisch ausgestaltet sein. Beispielsweise können die erste und zweite Klemme als Krokodilklemme ausgestaltet sein, wobei die jeweiligen Klemmschenkel bzw. die distalen Abschnitte der jeweiligen zwei Klemmschenkel der ersten und zweiten Klemme eine Zahnreihe aufweisen, die optional ineinandergreifen können. Alternativ können die erste und zweite Klemme auch als chirurgische Pinzette ausgestaltet sein, bei der sich zwei gegenüberliegende Dornen im geschlossenen Zustand berühren. Andere Ausgestaltungen der distalen Klemmabschnitte der ersten und zweiten Klemme sind erfindungsgemäß auch möglich.

Die erste und zweite Klammer können alternativ dazu auch unterschiedlich ausgestaltet sein. Hierbei können insbesondere der distale Klemmabschnitt der ersten und zweiten Klemme bzw. die distalen Abschnitte der Klemmschenkel der ersten und zweiten Klemme unterschiedlich ausgestaltet sein. Beispielsweise kann die erste Klemme als Krokodilklemme und die zweite Klemme als chirurgische Pinzette, oder umgekehrt, ausgestaltet sein.

Gemäß einer Ausführungsform der vorliegenden Erfindung liegen die erste und zweite Klemme bevorzugt in einem geschlossenen Handhabungszustand. In diesem Zustand berühren sich die jeweiligen Klemmschenkel bzw. die distalen Abschnitte der jeweiligen Klemmschenkel der ersten bzw. zweiten Klemme bevorzugt im jeweiligen distalen Klemmabschnitt; also in dem Abschnitt, in dem das Gewebe verklemmt werden soll. Der geschlossene Zustand der jeweiligen Klemmschenkel wird durch die jeweiligen Federn erreicht, die im geschlossenen Zustand der Klemme jeweils relaxiert sind. Umgekehrt sind die jeweiligen Federn der jeweiligen Klemmen zusammengedrückt bzw. auf Spannung, wenn sich die Klemmschenkel bzw. der distale Klemmabschnitt in einem offenen Zustand befinden; sich also die jeweiligen distalen Abschnitte der Klemmschenkel nicht berühren. Umgekehrt berühren sich die proximalen Abschnitte der Klemmschenkel in einem geschlossenen Handhabungszustand der chirurgischen Vorrichtung nicht, wohingegen sich die proximalen Abschnitte der Klemmschenkel in einem offenen Zustand berühren können.

Gemäß einer Ausführungsform der vorliegenden Erfindung handelt es sich bei der Feder der ersten und/oder zweiten Klemme bevorzugt um ein metallisches Bauteil, das sich elastisch verformen lässt. Optional kann die Feder eine isolierende Ummantelung aufweise, sodass sie nicht leitfähig ist. Bevorzugt liegt die Feder in einem relaxierten Handhabungszustand vor, der durch Ausübung von Druck auf den proximalen Betätigungsabschnitt in einen gestauchten Zustand, in dem die Feder auf Spannung ist, überführbar ist. Dies bietet den Vorteil, dass das Gewebe, welches auf Abstand gehalten werden soll, sicher in der chirurgischen Vorrichtung verklemmt werden kann.

Insbesondere bevorzugt ist es, wenn es sich bei der Feder um eine Schraubenfeder bzw. Schraubendruckfeder oder um eine Schenkelfeder handelt, wobei die Schenkelfeder besonders bevorzugt ist.

Gemäß einer Ausführungsform der vorliegenden Erfindung ist das Verbindungsstück "formstabil" ausgestaltet. Formstabil bedeutet in diesem Zusammenhang, dass sich das Verbindungsstück unter Aufwendung von Druck verformen lässt; das Verbindungsstück ist somit nicht starr ausgestaltet. Demnach weist das Verbindungsstück erfindungsgemäß eine eingeschränkte Flexibilität auf.

Gemäß einer Ausführungsform der vorliegenden Erfindung ist bevorzugt, wenn das Verbindungsstück an einem proximalen Abschnitt eines der beiden Klemmschenkels der jeweiligen Klemmen fixiert ist. Alternativ kann das Verbindungsstück auch im Bereich der Feder der jeweiligen Klammer fixiert werden.

Gemäß einer Ausführungsform der vorliegenden Erfindung kann die Fixierung des Verbindungsstücks beispielsweise durch Verkleben oder Verschweißen erreicht werden. Alternativ kann die Fixierung auch durch Verklemmen erreicht werden. Hierfür kann die jeweilige Klemme bzw. der jeweilige proximale Abschnitt eines Klemmschenkels eine Halterung aufweisen, in die das Verbindungsstück verklemmt oder hineingeschoben und anschließend verklemmt werden kann.

Gemäß einer Ausführungsform der vorliegenden Erfindung kann das längliche, formstabile Verbindungsstück bevorzugt als Draht ausgestaltet sein, wobei bevorzugt ist, wenn der Draht einen Durchmesser von 10 mm bis 50 mm aufweist, insbesondere von 15 mm bis 40 mm, insbesondere bevorzugt von 20 mm bis 30 mm. Sofern die erfindungsgemäße Vorrichtung innerhalb eines Roboter-unterstützenden/Roboter-assistierenden chirurgischen Eingriff eingesetzt wird, kann die der Draht des länglichen, formstabilen Verbindungsstücks bevorzugt einen Durchmesser von 1 mm bis 10 mm aufweisen, insbesondere von 1 mm bis 5 mm, insbesondere bevorzugt von 1 mm bis 3 mm.

Hierbei kann bevorzugt sein, dass der Draht aus einem der Folgenden Materialien besteht oder dieses enthält: rostfreier Stahl, insbesondere Edelstahl, Metalllegierungen, insbesondere solche aus Titan, Tantal, Messing, Kupfer, Silber. Darüber hinaus kann es bevorzugt sein, wenn es sich bei dem Draht um einen isolierten Draht handelt, der nicht leitfähig ausgestaltet ist. Als isolierendes Material ist ein Kunstsoff oder Silikon bevorzugt.

Gemäß einer bevorzugten Ausgestaltung der Erfindung ist die chirurgische Vorrichtung zur minimalinvasiven oder offen chirurgischen Einbringung in ein Gewebe ausgebildet und dimensioniert.

Diese Ausgestaltung hat unter anderem den Vorteil, dass mit der erfindungsgemäßen Vorrichtung ein chirurgisches Werkzeug bereitgestellt wird, das einen kleinen Durchmesser aufweist, so dass die Vorrichtung in einen kleinen Einschnitt oder eine kleine Körperöffnung des Patienten eingeführt werden kann.

Die chirurgische Vorrichtung gemäß der vorliegenden Erfindung ist besonders geeignet für minimalinvasive chirurgische Operationen oder andere medizinische Vorgänge, die durch kleine Einschnitte in der Körperwandung eines Patienten hindurch vorgenommen werden. Die chirurgische Vorrichtung gemäß der vorliegenden Erfindung kann jedoch neben dem Einsatz in der minimalinvasiven Chirurgie auch für andere medizinische Vorgänge verwendet werden, wie beispielsweise für externe Chirurgie oder die traditionelle offene Chirurgie, bei der große Einschnitte in die Körperwandung erfolgen. Demnach bietet die erfindungsgemäße chirurgische Vorrichtung ein breites Anwendungsgebiet innerhalb der Medizin.

Gemäß einer weiteren bevorzugten Ausgestaltung der Erfindung ist die chirurgische Vorrichtung zur chirurgischen, Roboter-unterstützenden Einbringung in ein Gewebe ausgebildet und dimensioniert.

Diese Ausgestaltung hat unter anderem den Vorteil, dass mit der erfindungsgemäßen Vorrichtung ein chirurgisches Werkzeug bereitgestellt wird, das gegenüber einem minimalinvasiven Werkzeug deutlich kleiner ausgestaltet sein kann, sodass der chirurgische Eingriff insgesamt zu weniger Komplikationen führt und sich die Patienten schneller erholen als nach traditionellen offenen Operationen, die einen großen Schnitt im Bauchraum erfordern. Demnach kann gemäß dieser Ausgestaltung die erfindungsgemäße Vorrichtung insgesamt einen kleinen Durchmesser aufweisen.

Gemäß einer weiteren bevorzugten Ausgestaltung der Erfindung sind der distale Klemmabschnitt der ersten Klemme und der distale Klemmabschnitt der zweiten Klemme durch Betätigen des jeweiligen proximalen Betätigungsabschnitts öffenbar.

Diese Ausgestaltung hat den Vorteil, dass es für einen zu behandelnden Arzt mittels eines einfachen Handgriff möglich ist, die jeweilige Klemme bzw. den jeweiligen distalen Klemmabschnitt zu öffnen.

Die Öffnung der jeweiligen Klemme bzw. des jeweiligen distalen Klemmabschnitts wird bevorzugt durch ausgeübten Druck auf den jeweiligen proximalen Betätigungsabschnitt erreicht. Dieser Druck kann beispielsweise mit einer Klammer oder durch zwei Finger des zu behandelnden Arztes auf den proximalen Betätigungsabschnitt ausgeübt werden. Durch den ausgeübten Druck wird die jeweilige Feder verformt, sodass die Feder von einem relaxierten Zustand in einen zusammengedrückten Zustand gebracht wird. Die Feder befindet sich anschließend unter Spannung. Fällt die Spannung weg, schließt sich der distale Klemmabschnitt der jeweiligen Klemmen aufgrund der Federwirkung, wodurch Gewebe sicher innerhalb der jeweiligen Klemmen verklemmt werden kann.

Gemäß einer weiteren bevorzugten Ausgestaltung weist die Feder der ersten und/oder zweiten Klemme eine Federkonstante von 0,005 bis 0,25 N/mm auf, insbesondere von 0,07 bis 0,15 N/mm, besonders bevorzugt von 0,1 bis 0,12 N/mm.

Gemäß dieser Ausgestaltung ist es besonders bevorzugt, wenn es sich bei der Feder um eine sogenannte Schenkelfeder handelt.

Diese Ausgestaltung hat den Vorteil, dass die Federkonstante bzw. die Federhärte derart gewählt ist, dass nur ein geringer Druck ausreichend ist, um die jeweilige Feder zusammen zu stauchen, um die distalen Klemmabschnitte zu öffnen. Dieser Druck kann beispielsweise mittels einer chirurgischen oder endoskopischen Klemme erreicht werden, sodass die erste und/oder zweite Klemme mittels einer chirurgischen oder endoskopischen Klemme geöffnet werden können.

Gemäß einer weiteren bevorzugten Ausgestaltung weist die Feder der ersten und/oder zweiten Klemme eine Federkonstante von 0,005 bis 0,025 N/mm auf, insbesondere von 0,007 bis 0,015 N/mm, besonders bevorzugt von 0,01 bis 0,012 N/mm.

Diese Ausgestaltung hat den Vorteil, dass die Federkonstante bzw. die Federhärte derart gewählt ist, dass ein sehr geringer Druck ausreichend ist, um die jeweilige Feder zusammen zu stauchen, um die distalen Klemmabschnitte zu öffnen. Dieser Druck kann beispielsweise mittels eines Greifarms eines Roboters, welcher innerhalb des chirurgischen Eingriffs eingesetzt wird, erreicht werden, sodass die erste und/oder zweite Klemme Roboter-assistierend geöffnet werden können.

Gemäß einer weiteren bevorzugten Ausgestaltung ist der distale Klemmabschnitt der ersten und/oder zweiten Klemme jeweils durch distale Abschnitte der Klemmschenkel ausgebildet ist, welche jeweils eine, zumindest eine Zahnreihe aufweisende Klemmbacke umfassen.

Gemäß einer Ausführungsform der vorliegenden Erfindung sind die jeweils zwei Klemmbacken derart ausgestaltet, dass das Gewebe innerhalb der Klemmbacken sicher verklemmt werden kann, ohne das Gewebe zu verletzen bzw. zu ruptieren. Hierfür sind die Klemmbacken über die Feder so miteinander verbunden, dass die Klemmbacken in einem geschlossenen Handhabungszustand aufeinander gedrückt werden.

Diese Ausgestaltung hat den Vorteil, dass das Gewebe sicher durch die Klemmbacken bzw. die zumindest eine Zahnreihe gehalten werden kann.

Gemäß einer Ausführungsform der vorliegenden Erfindung kann die Zahnreihe als einzelne hintereinanderliegende Zähne, die einen Bereich der Klemmbacken umgeben, oder als zick-zack förmige Fläche, die den gesamten Bereich der Klemmbacke ausfüllt, ausgestaltet sein. Gemäß einer Ausführungsform der vorliegenden Erfindung kann entweder ein Klemmschenkel oder jeweils beide Klemmschenkel einer Klemmbacke eine Zahnreihe aufweisen. Sofern beide Klemmschenkel einer Klemmbacke eine Zahnreihe aufweisen kann es bevorzugt sein, dass die Zähne der sich gegenüberliegenden Zahnreihen ineinandergreifen.

Gemäß einer weiteren bevorzugten Ausgestaltung der Erfindung ist der distale Klemmabschnitt der ersten und/oder zweiten Klemme jeweils durch distale Abschnitte der Klemmschenkel ausgebildet, welche jeweils einen endständigen Dorn umfassen, wobei die Dornen der Klemmschenkel aufeinander zeigend ausgebildet sind.

Gemäß einer Ausführungsform der vorliegenden Erfindung sind die Dornen bevorzugt derart ausgestaltet, dass sie das Gewebe, in welches sie geklemmt werden nicht verletzen. Hierfür kann es bevorzugt sein, wenn die endständigen bzw. distalen Dornen abgerundet sind. Diese Ausgestaltung hat den Vorteil, dass das Gewebe sicher durch die Dornen gehalten werden kann.

Gemäß einer weiteren bevorzugten Ausgestaltung ist der proximale Betätigungsabschnitt der ersten und/oder zweiten Klemme jeweils durch proximale Abschnitte der Klemmschenkel ausgebildet, die zum Betätigen durch eine chirurgische und/oder endoskopische Klemme ausgebildet und dimensioniert sind.

Gemäß dieser Ausgestaltung ist es vorteilhaft, wenn der proximale Betätigungsabschnitt Vertiefungen aufweist, in die eine chirurgische und/oder endoskopische Klemme hineingreifen kann. Diese Ausgestaltung hat den Vorteil, dass ein Öffnen und Schließen des distalen Klemmabschnitts der jeweiligen Klemmen erleichtert wird und dabei gleichzeitig ein ungewolltes Abrutschen der chirurgischen und/oder endoskopischen Klemme verhindert werden kann.

Gemäß einer weiteren bevorzugten Ausgestaltung ist die erste Klemme als Krokodilklemme mit zwei auf den Klemmschenkeln gegenüberliegenden Zahnreihen ausgestaltet, und die zweite Klemme ist als chirurgische Pinzette ausgestaltet, wobei die chirurgische Pinzette zwei gegenüberliegende Dornen aufweist.

Es hat sich gezeigt, dass sich diese Ausgestaltung der Klemmen besonders vorteilhaft zum Verklemmen von Gewebe eignet um beispielsweise Gewebe auf Abstand zu halten.

Gemäß einer weiteren bevorzugten Ausgestaltung weist die chirurgische Vorrichtung eine Länge (L) von 1,5 bis 15 cm auf, vorzugsweise eine Länge von 8 bis 10 cm ist, und noch bevorzugter eine Länge von 8 bis 9 cm.

Gemäß dieser Ausgestaltung wird die Länge der Vorrichtung ausgehend vom distalen Klemmabschnitt der ersten Klemme bis zum distalen Klemmabschnitt der zweiten Klemme gemessen. Es hat sich gezeigt, dass sich die genannten Längen besonders vorteilhaft bei chirurgischen Eingriffen eignen, um Gewebe auf Abstand zu halten und gleichzeitig das Gewebe sicher einzuklemmen. Ferner bieten die genannten Längen ein vereinfachtes Handling innerhalb des chirurgischen Eingriffs.

Gemäß einer weiteren bevorzugten Ausgestaltung weist die erste und/oder zweite Klemme eine Länge (L_{K1}, L_{K2}) von 0,5 bis 4 cm auf, wobei eine Länge von 2,5 bis 3,5 cm bevorzugt ist, wobei insbesondere eine Länge von 3 cm bevorzugt ist.

Gemäß dieser Ausgestaltung wird die Länge der ersten bzw. zweiten Klemme ausgehend vom distalen Klemmabschnitt bis zum proximalen Betätigungsabschnitt gemessen. Es hat sich gezeigt, dass sich die genannten Längen besonders vorteilhaft bei chirurgischen Eingriffen eignen um genügend Gewebe einklemmen zu können.

Gemäß einer weiteren bevorzugten Ausgestaltung beträgt der durch das Verbindungsstück gebildete Abstand zwischen der ersten Klemme und der zweiten Klemme von ca. 5 mm bis ca. 50 mm, wobei ein Abstand von ca. 20 mm bis 50 mm bevorzugt ist.

Gemäß dieser Ausgestaltung wird der Abstand zwischen der ersten und zweiten Klemme bzw. die Länge des Verbindungsstücks vom proximalen Betätigungsabschnitt der ersten Klemme bis zum proximalen Betätigungsabschnitt der zweiten Klemme gemessen. Es hat sich gezeigt, dass sich die genannten Längen besonders vorteilhaft bei chirurgischen Eingriffen eignen um Gewebe auf Abstand zu halten.

Gemäß einer weiteren bevorzugten Ausgestaltung weist die chirurgische Vorrichtung eine Länge (L) von 1,5 bis 7 cm auf, vorzugsweise eine Länge von 1,5 bis 5,5 cm ist, und noch bevorzugter eine Länge von 2 bis 4 cm.

Gemäß dieser Ausgestaltung wird die Länge der Vorrichtung ausgehend vom distalen Klemmabschnitt der ersten Klemme bis zum distalen Klemmabschnitt der zweiten Klemme gemessen. Es hat sich gezeigt, dass sich die genannten Längen besonders vorteilhaft bei chirurgischen, Roboter-unterstützenden Eingriffen eignen, um Gewebe auf Abstand zu halten und gleichzeitig das Gewebe sicher einzuklemmen.

Gemäß einer weiteren bevorzugten Ausgestaltung weist die erste und/oder zweite Klemme eine Länge (L_{K1}, L_{K2}) von 0,5 bis 2,5 cm auf, wobei eine Länge von 1 bis 1,5 cm bevorzugt ist, wobei insbesondere eine Länge von 1,5 cm bevorzugt ist.

Gemäß dieser Ausgestaltung wird die Länge der ersten bzw. zweiten Klemme ausgehend vom distalen Klemmabschnitt bis zum proximalen Betätigungsabschnitt gemessen. Es hat sich gezeigt, dass sich die genannten Längen besonders vorteilhaft bei chirurgischen, Roboter-unterstützenden Eingriffen eignen um genügend Gewebe einklemmen zu können.

Gemäß einer weiteren bevorzugten Ausgestaltung beträgt der durch das Verbindungsstück gebildete Abstand zwischen der ersten Klemme und der zweiten Klemme von ca. 5 mm bis ca. 25 mm.

Gemäß dieser Ausgestaltung wird der Abstand zwischen der ersten und zweiten Klemme bzw. die Länge des Verbindungsstücks vom proximalen Betätigungsabschnitt der ersten Klemme bis zum proximalen Betätigungsabschnitt der zweiten Klemme gemessen. Es hat sich gezeigt, dass sich die genannten Längen besonders vorteilhaft bei chirurgischen, Roboter-unterstützenden Eingriffen eignen um Gewebe auf Abstand zu halten.

Gemäß einer weiteren bevorzugten Ausgestaltung ist die chirurgische Vorrichtung, in einem Handhabungszustand, in dem die erste und die zweite Klemme geschlossen sind, zur Aufnahme in einer Einführhülse dimensioniert und ausgebildet, wobei die Einführhülse einen Durchmesser von 7 bis 15 mm, vorzugsweise einen Durchmesser von 8 bis 10 mm, aufweist

Diese Ausgestaltung hat den Vorteil, das die chirurgische Vorrichtung derart ausgestaltet ist, um innerhalb eines minimalinvasiven Eingriff durch eine Einführhülse eingeführt und wieder herausgeführt zu werden. Um durch die Einführhülse geführt zu werden, weist die chirurgische Vorrichtung demnach bevorzugt einen kleineren Durchmesser als die Einführhülse auf; beispielsweise von 6 bis 14 mm, vorzugsweise einen Durchmesser von 7 bis 9 mm, um nicht in der Einführhülse zu verklemmen bzw. stecken zu bleiben.

Gemäß einer Ausführungsform der vorliegenden Erfindung kann es sich bei der Einführhülse um einen Tubus handeln, durch den bei einem minimalinvasiven Eingriff chirurgische Instrumente in die Körperhöhle geführt werden können.

Gemäß einer weiteren bevorzugten Ausgestaltung ist die erste und/oder zweite Klemme und/oder das Verbindungsstück aus einem nicht oder nur gering Elektrizitäts-leitfähigem Material hergestellt, oder mit diesem beschichtet, wobei das Material ausgewählt ist aus Kunststoff, Silikon und Keramik.

Diese Ausgestaltung hat den Vorteil, dass Kurzschlüsse sowie ungewollte Verletzungen am Gewebe während eines chirurgischen Eingriffs vermieden werden können. Zu solchen Kurzschlüssen/Verletzungen kann es kommen, wenn elektrisch leitfähige Instrumente während einem chirurgischen Einsatz eingesetzt werden.

Gemäß einer weiteren bevorzugten Ausgestaltung weist die chirurgische Vorrichtung eine lösbare Markierung auf.

Gemäß einer Ausführungsform der vorliegenden Erfindung kann es sich bei der lösbaren Markierung um einen sogenannten Klebe-Tag handeln. Mit der lösbaren Markierung ist es für einen zu behandelnden Arzt nachvollziehbar ob sich die erfindungsgemäße Vorrichtung noch im zu behandelnden Körper befindet oder bereits aus dem Körper entfernt wurde. Hierfür kann der zu behandelnde Arzt die Markierung unmittelbar vor der Verwendung innerhalb einer Körperhöhle von der Vorrichtung ablösen und sicher verwahren, bevor er die erfindungsgemäße Vorrichtung verwendet. Nach Beendigung des chirurgischen Eingriffs kann der zu behandelnde Arzt die Anzahl der Markierungen mit der Anzahl der chirurgischen Vorrichtungen abgleichen, sodass kein Instrument im Körper ungewollt vergessen wird.

Weitere Vorteile ergeben sich aus den Figuren der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Nachstehend wird die Erfindung anhand der aufgeführten Beispiele und Figuren beispielhaft näher erläutert. Es zeigen:
- Figur 1: eine Seitenansicht einer schematischen Darstellung einer ersten Ausführungsform der chirurgischen Vorrichtung;
- Figur 2: eine Vorderansicht einer schematischen Darstellung der ersten Ausführungsform der chirurgischen Vorrichtung innerhalb einer Einführhülse;
- Figur 3: eine Seitenansicht einer schematischen Darstellung einer ersten Ausführungsform der ersten Klemme;
- Figur 4: eine Seitenansicht einer schematischen Darstellung einer zweiten Ausführungsform der chirurgischen Vorrichtung mit einer Einführhülse und einer endoskopischen Klemme;
- Figur 5: eine Seitenansicht einer schematischen Darstellung einer ersten Ausführungsform der zweiten Klemme; und
- Figur 6: eine Seitenansicht einer schematischen Darstellung einer dritten Ausführungsform der chirurgischen Vorrichtung mit einer chirurgischen Klemme.

Funktionsäquivalente Elemente werden in allen Figuren, auch bei unterschiedlichen Ausführungsformen, mit den gleichen Bezugszeichen gekennzeichnet.

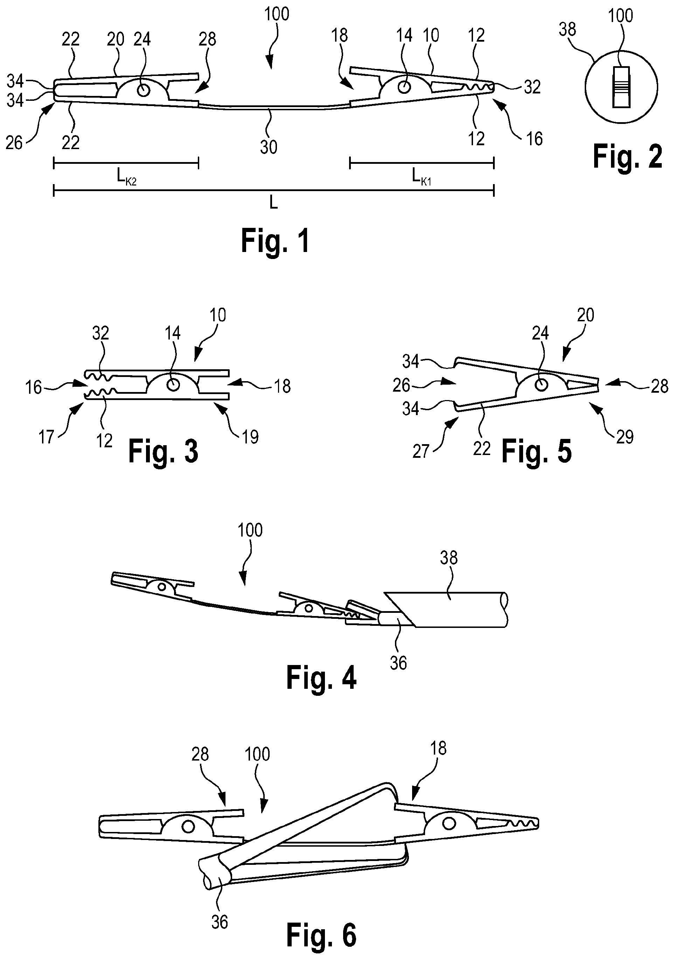

Fig. 1 zeigt eine Seitenansicht einer schematischen Darstellung einer ersten Ausführungsform der chirurgischen Vorrichtung 100. Die chirurgische Vorrichtung 100 weist eine erste Klemme 10, eine zweite Klemme 20 und ein längliches formstabiles Verbindungsstück 30 auf. Die beiden Klemmen 10 und 20 liegen in einem geschlossenen Handlungszustand vor. Die Länge der chirurgischen Vorrichtung 100 wird mit L angegeben. Die Länge der ersten Klammer 10 wird mit L_{K1} angegeben, wohingegen die Länge der zweiten Klammer 20 wird mit L_{K2} angegeben ist.

Die erste Klemme 10 ist mit zwei sich gegenüberliegenden Klemmschenkeln 12 ausgestaltet, wobei die beiden Klemmschenkel 12 durch eine gelenkartig gelagerte Feder 14 zusammengehalten werden. Die Feder 14 als solche ist in Fig. 1 nicht dargestellt, sondern lediglich das Gelenk über das sich die zwei Klemmschenkel 12 gegeneinander auslenkend bewegen lassen können. Die erste Klemme 10 ist in einem geschlossenen Handlungszustand gezeigt. Hierfür berühren sich die Klemmschenkel 12 in einem distalen Klemmabschnitt 16 bzw. die beiden distalen Abschnitte 17 der Klemmschenkel 12.

Die erste Klemme 10 weist einen distalen Klemmabschnitt 16 auf, der zum Einklemmen und Halten von Gewebe ausgebildet ist. Die erste Klemme 10 weist ferner einen proximalen Betätigungsabschnitt 18 zum Öffnen und Schließen des distalen Klemmabschnitts16 auf. Der distale Klemmabschnitt 16 wird insbesondere durch den distalen Abschnitt17 der Klemmschenkel 12 gebildet, wohingegen der proximalen Betätigungsabschnitt 18 durch den proximalen Abschnitt 19 der Klemmschenkel 12 gebildet wird.

In der Fig. 1 ist die erste Klemme 10 als Krokodilklemme 10 ausgestaltet. Die Krokodilklemme 10 weist zwei gegenüberliegende Zahnreihen 32 auf, die im geschlossenen Zustand ineinandergreifen. Bevorzugt sind die Zahnreihen 32 abgerundet ausgestaltet, damit das eingeklemmte Gewebe nicht verletzt/ruptiert wird.

Die zweite Klemme 20 ist mit zwei sich gegenüberliegenden Klemmschenkeln 22 ausgestaltet, wobei die beiden Klemmschenkel 22 durch eine gelenkartig gelagerte Feder 24 zusammengehalten werden. Die Feder 24 als solche ist in Fig. 1 nicht dargestellt, sondern lediglich das Gelenk über das sich die zwei Klemmschenkel 22 gegeneinander auslenkend bewegen lassen können. Die zweite Klemme 20 ist in einem geschlossenen Handlungszustand gezeigt. Hierfür berühren sich die Klemmschenkel 22 in einem distalen Klemmabschnitt 26 bzw. die beiden distalen Abschnitte 27 der Klemmschenkel 22.

Die zweite Klemme 20 weist einen distalen Klemmabschnitt 26 auf, der zum Einklemmen und Halten von Gewebe ausgebildet ist. Die zweite Klemme 20 weist ferner einen proximalen Betätigungsabschnitt 28 zum Öffnen und Schließen des distalen Klemmabschnitts26 auf. Der distale Klemmabschnitt 26 wird insbesondere durch den distalen Abschnitt 27 der Klemmschenkel 22 gebildet, wohingegen der proximalen Betätigungsabschnitt 18 durch den proximalen Abschnitt 19 der Klemmschenkel 22 gebildet wird.

In der Fig. 1 ist die zweite Klemme 20 als chirurgische Pinzette 20 ausgestaltet. Die chirurgische Pinzette 20 weist zwei gegenüberliegende Dornen 34 auf, die im geschlossenen Zustand aufeinanderliegen bzw. sich an den Dornenenden berühren. Bevorzugt sind die Dornen 34 abgerundet ausgestaltet, damit das eingeklemmte Gewebe nicht verletzt wird.

In Fig. 1 sind die erste und zweite Klemme 10, 20 unterschiedlich ausgestaltet; als Krokodilklemme 10 bzw. als chirurgische Pinzette 20. In einer nicht dargestellten Ausführungsform können die beiden Klemmen 10, 20 auch jeweils identisch ausgestaltet sein.

Das längliche formstabile Verbindungsstück 30 ist in Fig. 1 als Draht dargestellt, der am proximalen Betätigungsabschnitt 18 der ersten Klemme 10 und am proximalen Betätigungsabschnitt 28 der zweiten Klemme 20 fixiert ist, um einen stabilen Abstand zwischen der ersten und zweiten Klemme 10 und 20 zu erreichen. In der in Fig. 1 gezeigten Ausgestaltung weist jeweils einer der beiden Klemmschenkel 12 bzw. 22 der ersten bzw. zweiten Klemme 10, 20 an seinem proximalen Abschnitt eine Fixierungsstelle auf, die als Zylinder ausgestaltet sind, in die das Verbindungsstück hineingesteckt und fixiert werden kann. Gemäß einer Ausführungsform der vorliegenden Erfindung kann das Verbindungsstück 30 auch anders fixiert werden, beispielsweise im Bereich der Feder 14, 24.

Fig. 2 zeigt eine Vorderansicht einer schematischen Darstellung der ersten Ausführungsform der chirurgischen Vorrichtung 100 innerhalb einer Einführhülse 38. Wie in dieser Fig. 2 gut zu erkennen ist, ist die chirurgische Vorrichtung 100 bevorzugt zur minimalinvasiven Einbringung in ein Gewebe ausgebildet und dimensioniert, sodass die chirurgische Vorrichtung 100 durch eine Einführhülse 38 geführt werden kann.

In Fig. 2 ist die Einführhülse 38 als bloßer Zylinder dargestellt. Gemäß einer Ausführungsform der vorliegenden Erfindung kann es sich bei der Einführhülse um ein Trokar oder um ein Tubus handeln, über den nicht nur die erfindungsgemäße Vorrichtung 100 sondern auch weitere medizinische Instrumente geführt werden können.

Fig. 3 zeigt eine Seitenansicht einer schematischen Darstellung einer ersten Ausführungsform der ersten Klemme 10. Die erste Klemme 10 ist in dieser Ausführungsform als Krokodilklemme 10 ausgestaltet; analog zu Fig. 1. Gemäß einer Ausführungsform der vorliegenden Erfindung kann die erste Klemme 10 auch als eine chirurgische Klemme 20 ausgestaltet sein, wie sie in Fig. 5 beispielhaft gezeigt ist.

Die Klemme 10 ist in Fig. 3 zumindest teilweise geöffnet dargestellt, sodass die beiden Klemmschenkel 12 parallel zueinander liegen. In diesem Zustand berühren sich die jeweiligen Klemmschenkel 12 bzw. die distalen Abschnitte 17 der jeweiligen Klemmschenkel 12 der ersten Klemme 10 im distalen Klemmabschnitt 16 nicht; also in dem Abschnitt, in dem das Gewebe verklemmt werden soll.

Fig. 4 zeigt eine Seitenansicht einer schematischen Darstellung einer zweiten Ausführungsform der chirurgischen Vorrichtung 100 mit einer Einführhülse 38 und einer endoskopischen Klemme 36. Diese Figur zeigt an, wie die chirurgische Vorrichtung 100 durch eine Einführhülse geführt werden kann. Die Einführhülse 36 ist in dieser Figur als Tubus 36 gezeichnet. Die Einführhülse ist insbesondere bei endoskopischen Behandlungen bevorzugt. Bei offen-chirurgischen Behandlungen kann eine Einführhülse überflüssig sein.

Beispielsweise kann die chirurgische Vorrichtung 100 mittels einer endoskopischen Klemme 36 in eine Körperöffnung eines Patienten geführt werden. Alternativ kann die chirurgische Vorrichtung auch mit jedem andern geeigneten chirurgischen Werkzeug zur behandelnden Stelle geführt werden. Je nachdem ob es sich um eine endoskopische oder offen-chirurgische Behandlung handelt können unterschiedliche Einführ-Klemmen vorteilhaft sein.

Fig. 5 zeigt eine Seitenansicht einer schematischen Darstellung einer ersten Ausführungsform der zweiten Klemme 20. Die zweite Klemme 20 ist in dieser Ausführungsform als chirurgische Pinzette 20 ausgestaltet; analog zu Fig. 1; analog zu Fig. 1. Gemäß einer Ausführungsform der vorliegenden Erfindung kann die zweite Klemme 20 auch als eine chirurgische Klemme 20 ausgestaltet sein, wie sie in Fig. 3 beispielhaft gezeigt ist. Die Klemme 20 ist in Fig. 5 vollständig geöffnet dargestellt, sodass sich die beiden proximalen Abschnitte 29 der Klemmschenkel 22 im proximalen Betätigungsabschnitt 18 berühren, wohingegen der distale Klemmabschnitt 26 vollständig geöffnet vorliegt.

Fig. 6 zeigt eine Seitenansicht einer schematischen Darstellung einer dritten Ausführungsform der chirurgischen Vorrichtung 100 mit einer chirurgischen Klemme 36. Zu sehen ist die Funktionsweise des proximalen Betätigungsabschnitts 18, der zum Öffnen und Schließen des distalen Klemmabschnitts 16 ausgebildet ist. Gezeigt wird die Funktionsweise an der ersten Klammer 10 der chirurgischen Vorrichtung 100. Gemäß einer Ausführungsform der vorliegenden Erfindung kann die zweite Klemme 20 analog durch die chirurgische Klemme 36 betätigt werden.

Durch ausgeübten Druck der chirurgischen Klemme 36 auf den proximalen Bereich 18 der Klemmschenkel 12, werden die beiden proximalen Abschnitte 19 der Klemmschenkel12 gegeneinander gedrückt, sodass die Feder 14 zusammengestaucht wird und sich die distalen Abschnitte 17 der Klemmschenkel 12 öffnen zum Einklemmen des Gewebes. Wird die chirurgische Klemme 36 von der chirurgischen Vorrichtung gelöst, schließt sich die Klemme 10 von selbst aufgrund der Federwirkung, sodass das Gewebe innerhalb der zwei Klemmschenkel 12 eingeklemmt wird.

Um ein sicheres Öffnen der Klemmen 10 und 20 gewährleisten zu können, ist es von Vorteil, wenn der proximale Betätigungsabschnitt 18 Vertiefungen aufweist, in die eine chirurgische und/oder endoskopische Klemme 36 hineingreifen kann (nicht gezeigt). Diese Ausgestaltung hat den Vorteil, dass ein Öffnen und Schließen des distalen Klemmabschnitts 16 der jeweiligen Klemmen 10, 20 erleichtert wird und dabei gleichzeitig ein ungewolltes Abrutschen der chirurgischen und/oder endoskopischen Klemme 36 verhindert werden kann.

## Patentansprüche

1. Chirurgische Vorrichtung (100) zur Verklemmung in Gewebe, wobei die chirurgische Vorrichtung (100) Folgendes aufweist:
- eine erste Klemme (10) mit zwei Klemmschenkeln (12) und einer die Klemmschenkel (12) zusammenhaltende, gelenkartig gelagerte Feder (14), wobei die erste Klemme (10) einen distalen Klemmabschnitt (16) aufweist, der zum Einklemmen und Halten von Gewebe ausgebildet ist, und einem proximalen Betätigungsabschnitt (18), der zum Öffnen und Schließen des distalen Klemmabschnitts (16) der ersten Klemme (10) ausgebildet ist,
- eine zweite Klemme (20) mit zwei Klemmschenkeln (22) und einer die Klemmschenkel (22) zusammenhaltende, gelenkartig gelagerte Feder (24), wobei die zweite Klemme (20) einen distalen Klemmabschnitt (26) aufweist, der zum Einklemmen und Halten von Gewebe ausgebildet ist, und einen proximalen Betätigungsabschnitt (28), der zum Öffnen und Schließen des distalen Klemmabschnitts (26) der zweiten Klemme (20) ausgebildet ist, **dadurch gekennzeichnet, dass** die Vorrichtung ferner aufweist:
- ein längliches formstabiles Verbindungsstück (30) zum Verbinden der ersten und zweiten Klemme (10, 20), wobei das Verbindungsstück (30) am proximalen Betätigungsabschnitt (18) der ersten Klemme (10) und am proximalen Betätigungsabschnitt (28) der zweiten Klemme (20) zur Ausbildung eines stabilen Abstands zwischen der ersten Klemme (10) und der zweiten Klemme (20) fixiert ist.

2. Chirurgische Vorrichtung (100) nach Anspruch 1, wobei die chirurgische Vorrichtung (100) zur minimalinvasiven oder offen chirurgischen Einbringung in ein Gewebe ausgebildet und dimensioniert ist.

3. Chirurgische Vorrichtung (100) nach Anspruch 1 oder 2, wobei der distale Klemmabschnitt (16) der ersten Klemme und der distale Klemmabschnitt (26) der zweiten Klemme (20) durch Betätigen des jeweiligen proximalen Betätigungsabschnitts (18, 28) öffenbar sind.

4. Chirurgische Vorrichtung (100) nach einem der vorstehenden Ansprüche, wobei die Feder (14, 24) der ersten und/oder zweiten Klemme (10, 20) eine Federkonstante von 0,005 bis 0,25 N/mm aufweist, insbesondere von 0,07 bis 0,15 N/mm, besonders bevorzugt von 0,1 bis 0,12 N/mm.

5. Chirurgische Vorrichtung (100) nach einem der vorstehenden Ansprüche, wobei der distale Klemmabschnitt (16, 26) der ersten und/oder zweiten Klemme (10, 20) jeweils durch distale Abschnitte (17) der Klemmschenkel (12) ausgebildet ist, welche jeweils eine, zumindest eine Zahnreihe (32) aufweisende Klemmbacke umfassen.

6. Chirurgische Vorrichtung (100) nach einem der vorstehenden Ansprüche, wobei der distale Klemmabschnitt (16, 26) der ersten und/oder zweiten Klemme (10, 20) jeweils durch distale Abschnitte (27) der Klemmschenkel (22) ausgebildet ist, welche jeweils einen endständigen Dorn (34) umfassen, wobei die Dornen (34) der Klemmschenkel (22) aufeinander zeigend ausgebildet sind.

7. Chirurgische Vorrichtung (100) nach einem der vorstehenden Ansprüche, wobei der proximale Betätigungsabschnitt (18, 28) der ersten und/oder zweiten Klemme (10, 20) jeweils durch proximale Abschnitte (19, 29) der Klemmschenkel (12, 22) ausgebildet ist, die zum Betätigen durch eine chirurgische und/oder endoskopische Klemme (36) ausgebildet und dimensioniert sind.

8. Chirurgische Vorrichtung (100) nach einem der vorherigen Ansprüche, wobei die erste Klemme (10) als Krokodilklemme (10) mit zwei auf den Klemmschenkeln (12) gegenüberliegenden Zahnreihen (32) ausgestaltet ist, und wobei die zweite (20) Klemme als chirurgische Pinzette (20) ausgestaltet ist, wobei die chirurgische Pinzette (20) zwei gegenüberliegende Dornen (34) aufweist.

9. Chirurgische Vorrichtung (100) nach einem der vorherigen Ansprüche, wobei die chirurgische Vorrichtung (100) eine Länge (L) von 1,5 bis 15 cm aufweist, vorzugsweise eine Länge von 8 bis 10 cm ist, und noch bevorzugter eine Länge von 8 bis 9 cm.

10. Chirurgische Vorrichtung (100) nach einem der vorherigen Ansprüche, wobei die erste und/oder zweite Klemme (10, 20) eine Länge (L_{K1}, L_{K2}) von 0,5 bis 4 cm aufweist, wobei eine Länge von 2,5 bis 3,5 cm bevorzugt ist, wobei insbesondere eine Länge von 3 cm bevorzugt ist.

11. Chirurgische Vorrichtung (100) nach einem der vorherigen Ansprüche, wobei der durch das Verbindungsstück (30) gebildete Abstand zwischen der ersten Klemme (10) und der zweiten Klemme (20) von ca. 5 mm bis ca. 50 mm beträgt, wobei ein Abstand von ca. 20 mm bis 50 mm bevorzugt ist.

12. Chirurgische Vorrichtung (100) nach einem der vorherigen Ansprüche, wobei die chirurgische Vorrichtung (100), in einem Handhabungszustand, in dem die erste und die zweite Klemme (10, 20) geschlossen sind, zur Aufnahme in einer Einführhülse (38) dimensioniert und ausgebildet ist, wobei die Einführhülse (38) einen Durchmesser von 7 bis 15 mm, vorzugsweise einen Durchmesser von 8 bis 10 mm, aufweist.

13. Chirurgische Vorrichtung (100) nach einem der vorherigen Ansprüche, wobei die erste und/oder zweite Klemme (10, 20) und/oder das Verbindungsstück (30) aus einem nicht oder nur gering Elektrizitäts-leitfähigem Material hergestellt ist, oder mit diesem beschichtet ist, wobei das Material ausgewählt ist aus Kunststoff, Silikon und Keramik.

14. Chirurgische Vorrichtung (100) nach einem der vorherigen Ansprüche, wobei die chirurgische Vorrichtung (100) eine lösbare Markierung aufweist.

## Claims

1. A surgical device (100) for clamping in tissue, wherein the surgical device (100) comprises
- a first clamp (10) comprising two clamp-legs (12) and a hinge-like articulated spring (14) holding the clamp-legs (12) together, wherein the first clamp (10) comprises a distal clamping portion (16) configured for clamping and holding tissue, and a proximal actuating portion (18) configured for opening and closing the distal clamping portion (16) of the first clamp (10),
- a second clamp (20) comprising two clamp-legs (22) and a hinge-like articulated spring (24) holding the clamp-legs (22) together, wherein the second clamp (20) comprises a distal clamping portion (26) configured for clamping and holding tissue and a proximal actuating portion (28) configured for opening and closing the distal clamping portion (26) of the second clamp (20), **characterized in that** the device further comprises
- an elongated dimensionally stable connector (30) for connecting the first and second clamps (10, 20), wherein the connector (30) is fixed to the proximal actuating portion (18) of the first clamp (10) and to the proximal actuating portion (28) of the second clamp (20) to form a stable distance between the first clamp (10) and the second clamp (20).

2. The surgical device (100) of claim 1, wherein the surgical device (100) is configured for and dimensioned for minimally invasive or open surgical insertion into a tissue.

3. The surgical device (100) of claim 1 or 2, further being designed such, that by actuation of the respective proximal actuating portion (18, 28) the distal clamping portion (16) of the first clamp (10) and the distal clamping portion (26) of the second clamp (20) can be opened.

4. The surgical device (100) of any of the preceding claims, wherein the spring (14, 24) of the first and/or second clamp comprises a spring constant of 0.005 to 0.25 N/mm, preferably of 0.07 to 0.15 N/mm, more preferably of 0.1 to 0.12 N/mm.

5. The surgical device (100) of any of the preceding claims, wherein the distal clamping portion (16, 26) of the first and/or second clamp (10, 20) is respectively formed through distal portions (17) of the clamp-legs (12), which each comprise a clamping jaw comprising at least one row of teeth (32).

6. The surgical device (100) of any of the preceding claims, wherein the distal clamping portion (16, 26) of the first and/or second clamp (10, 20) is respectively formed through distal portions (27) of the clamp-legs (22), which each comprise a terminal spike (34), wherein the spikes (34) of the clamp-legs (22) point towards each other.

7. The surgical device (100) of any of the preceding claims, wherein the proximal actuating portion (18, 28) of the first and/or second clamp (10, 20) is respectively formed by proximal portions (19, 29) of the clamp-legs (12, 22), which are configured and dimensioned for actuation by a surgical and/or endoscopic clamp (36).

8. The surgical device (100) of any of the preceding claims, wherein the first clamp (10) is embodied as a crocodile clamp comprising two opposing rows (32) of teeth on the clamp-legs (12), and wherein the second clamp (12) is provided as surgical forceps, wherein the surgical forceps comprises two opposing spikes (34).

9. The surgical device (100) of any of the preceding claims, wherein the surgical device (100) comprises a length (L) of 1.5 to 15 cm, preferably a length of 8 to 10 cm, and more preferably a length of 8 to 9 cm.

10. The surgical device (100) of any of the preceding claims, wherein the first and/or second clamp (10, 20) comprises a length (L_{K1}, L_{K2}) of 0.5 to 4 cm, preferably a length of 2.5 to 3.5 cm, more preferably a length of 3 cm.

11. The surgical device (100) of any of the preceding claims, wherein the distance between the first clamp (10) and the second clamp (20) as defined through the connector (30) is from about 5 mm to about 50 mm, preferably from about 20 mm to 50 mm.

12. The surgical device (100) of any of the preceding claims, wherein the surgical device (100), in a handling state in which the first and second clamps (10, 20) are closed, is dimensioned and configured for being accommodated in an introduction sleeve (38), wherein the introduction sleeve (38) comprises a diameter of 7 to 15 mm, preferably a diameter of 8 to 10 mm.

13. The surgical device (100) of any of the preceding claims, wherein the first and/or second clamp (10, 20) and/or the connector (30) is made of or coated with a material that is not or only slightly electrically conductive, wherein the material is selected from plastic, silicone and ceramic.

14. The surgical device (100) of any of the preceding claims, wherein the surgical device (100) comprises a removable label.

## Revendications

1. Dispositif chirurgical (100) permettant le pincement dans un tissu, dans lequel le dispositif chirurgical (100) présente ce qui suit :
- une première pince (10) comportant deux branches de pincement (12) et un ressort (14) monté de manière à être articulé et maintenant les branches de pincement (12) ensemble, dans lequel la première pince (10) présente une section de pincement distale (16) réalisée pour pincer et maintenir un tissu, et une section d'actionnement proximale (18) réalisée pour ouvrir et fermer la section de pincement distale (16) de la première pince (10),
- une seconde pince (20) comportant deux branches de pincement (22) et un ressort (24) monté de manière à être articulé et maintenant les branches de pincement (22) ensemble, dans lequel la seconde pince (20) présente une section de pincement distale (26) réalisée pour pincer et maintenir un tissu, et une section d'actionnement proximale (28) réalisée pour ouvrir et fermer la section de pincement distale (26) de la seconde pince (20), **caractérisé en ce que** le dispositif présente en outre :
- une pièce de liaison (30) allongée et indéformable pour la liaison de la première et de la seconde pince (10, 20), dans lequel la pièce de liaison (30) est fixée à la section d'actionnement proximale (18) de la première pince (10) et à la section d'actionnement proximale (28) de la seconde pince (20) pour la formation d'un espacement stable entre la première pince (10) et la seconde pince (20).

2. Dispositif chirurgical (100) selon la revendication 1, dans lequel le dispositif chirurgical (100) est réalisé et dimensionné pour l'insertion chirurgicale mini-invasive ou à ciel ouvert dans un tissu.

3. Dispositif chirurgical (100) selon la revendication 1 ou 2, dans lequel la section de pincement distale (16) de la première pince et la section de pincement distale (26) de la seconde pince (20) peuvent être ouvertes en actionnant la section d'actionnement proximale (18, 28) respective.

4. Dispositif chirurgical (100) selon l'une des revendications précédentes, dans lequel le ressort (14, 24) de la première et/ou de la seconde pince (10, 20) présente une flexibilité de ressort allant de 0,005 à 0,25 N/mm, en particulier de 0,07 à 0,15 N/mm, de manière particulièrement préférée de 0,1 à 0,12 N/mm.

5. Dispositif chirurgical (100) selon l'une des revendications précédentes, dans lequel la section de pincement distale (16, 26) de la première et/ou de la seconde pince (10, 20) est respectivement réalisée par des sections distales (17) des branches de pincement (12) comprenant respectivement une mâchoire de pincement présentant au moins une rangée de dents (32).

6. Dispositif chirurgical (100) selon l'une des revendications précédentes, dans lequel la section de pincement distale (16, 26) de la première et/ou de la seconde pince (10, 20) est respectivement réalisée par des sections distales (27) des branches de pincement (22) comprenant respectivement une épine (34) terminale, dans lequel les épines (34) des branches de pincement (22) sont réalisées de manière à se faire face.

7. Dispositif chirurgical (100) selon l'une des revendications précédentes, dans lequel la section d'actionnement proximale (18, 28) de la première et/ou de la seconde pince (10, 20) est respectivement réalisée par des sections proximales (19, 29) des branches de pincement (12, 22) réalisées et dimensionnées pour être actionnées par une pince chirurgicale et/ou endoscopique (36).

8. Dispositif chirurgical (100) selon l'une des revendications précédentes, dans lequel la première pince (10) est conçue sous forme de pince crocodile (10) comportant deux rangées de dents (32) opposées sur les branches de pincement (12), et dans lequel la seconde pince (20) est conçue sous forme de pincette chirurgicale (20), dans lequel la pincette chirurgicale (20) présente deux épines (34) opposées.

9. Dispositif chirurgical (100) selon l'une des revendications précédentes, dans lequel le dispositif chirurgical (100) présente une longueur (L) allant de 1,5 à 15 cm, de préférence une longueur allant de 8 à 10 cm, et de manière encore plus préférée une longueur allant de 8 à 9 cm.

10. Dispositif chirurgical (100) selon l'une des revendications précédentes, dans lequel la première et/ou la seconde pince (10, 20) présentent une longueur (L_{K1}, L_{K2}) allant de 0,5 à 4 cm, dans lequel une longueur allant de 2,5 à 3,5 cm est préférée, dans lequel, en particulier, une longueur de 3 cm est préférée.

11. Dispositif chirurgical (100) selon l'une des revendications précédentes, dans lequel l'espacement formé par la pièce de liaison (30) entre la première pince (10) et la seconde pince (20) va d'environ 5 mm à environ 50 mm, dans lequel un espacement allant d'environ 20 mm à 50 mm est préféré.

12. Dispositif chirurgical (100) selon l'une des revendications précédentes, dans lequel le dispositif chirurgical (100), dans un état de manipulation dans lequel la première et la seconde pince (10, 20) sont fermées, est dimensionné et réalisé pour être reçu dans une gaine d'introduction (38), dans lequel la gaine d'introduction (38) présente un diamètre allant de 7 à 15 mm, de préférence un diamètre allant de 8 à 10 mm.

13. Dispositif chirurgical (100) selon l'une des revendications précédentes, dans lequel la première et/ou la seconde pince (10, 20) et/ou la pièce de liaison (30) sont fabriquées à partir d'un matériau non électroconducteur, ou seulement faiblement, ou sont revêtues dudit matériau, dans lequel le matériau est choisi parmi le plastique, le silicone et la céramique.

14. Dispositif chirurgical (100) selon l'une des revendications précédentes, dans lequel le dispositif chirurgical (100) présente un marquage amovible.
